# EUROPEAN PATENT APPLICATION

(11) **EP 1 419 820 A1**
(43) Date of publication of application: **19.05.2004**
(21) Application number: 02079749.4
(22) Date of filing: 14.11.2002
(51) Int. Cl.: B01L 3/14, B01F 11/00, G01N 33/543

(54) **Method, system and reaction vessel for processing a biological sample contained in a liquid**

(71) Applicant: F. HOFFMANN-LA ROCHE AG, 4070 Basel (CH); Roche Diagnostics GmbH, 68305 Mannheim (DE)
(72) Inventor: Hutter, Roland Christof, 6300 Zug (CH); Schmid, Karl Anton Josef, 8808 Pfäffikon (CH)
(74) Representative: Ventocilla, Abraham

(57) **Abstract**

Method for processing a biological sample contained in a liquid. The method comprises
(a) introducing said liquid into a chamber (17) of a reaction vessel (11) which comprises a tubular body (12) which has a bottom wall (13), an upper opening (14) and side walls (15, 16) which extend between said bottom wall (13) and said upper opening (14), said bottom wall (13) and said side walls (15, 16) forming said chamber (17), and a chip shaped carrier (21) having an active surface which is formed by an array of biological polymers, said active surface being accessible to liquid contained in said chamber (17),
   said chip shaped carrier (21) being located in an opening (31) of a side wall (16) of said tubular body (12) or on the inner surface of said side wall (16) or in a recess formed in the inner surface of said side wall (16),
(b) positioning said reaction vessel (11) in a vessel holder, said positioning being effected before or after introduction of said liquid into said chamber (17), and
(c) moving said vessel holder along a predetermined trajectory for causing a relative motion of the liquid contained in said chamber (17) with respect to said active surface of said chip shaped carrier (21).

## Description

### FIELD OF THE INVENTION

The invention concerns a method according to the preamble of claim 1.

The invention further concerns a system according to the preamble of claim 5.

The invention further concerns a reaction vessel according to the preamble of claim 9.

### BACKGROUND OF THE INVENTION

There are known disposables cartridges containing a chip shaped carrier having an active surface for the analysis of biological samples and in particular of nucleic acids contained in liquid samples. A cartridge of this kind is described in European Patent Application EP 1224976 A1 which corresponds to U.S. Patent Application 10/033,424.

In known devices of this kind, the chip shaped carrier is so arranged within a process chamber of the cartridge that the active surface of the chip shaped carrier is nearly coplanar with an inner surface of the process chamber. A process chamber of this kind is a flow-through cell which has e.g. a rectangular cross-section, a width in a range going from 0.5 to 20 millimeter and a depth in a range going from 0.05 to 1 millimeter. A process chamber having these dimensions is described in U.S. Patent Specification No. 6,197,595.

Such a cartridge has an inlet and an outlet which allow introduction respectively removal of a liquid sample to be analyzed into respectively from the above-mentioned chamber.

In order to provide the necessary contact between the liquid sample to be analyzed and the reactants on the active surface of the chip shaped carrier, a relative motion between sample and active surface is provided e.g. by an oscillatory movement of the cartridge as described in European Patent Application EP 1224976 A1 or by a pumping action that moves the liquid sample back and forth within the process chamber.

Within the context of the instant invention a chip shaped carrier is a substrate, in particular a glass or silicon chip of e.g. squared shape having a thickness of e.g. 0.7 or 1.0 millimeter and a so called active surface, which is a surface coated with an array of biological polymers, e.g. an array of different snippets of DNA, e.g. DNA oligonucleotide probes, located at known positions on that surface. Those snippets of DNA serve as probes for detecting DNA fragments with a complementary DNA sequence. Biological polymers are e.g. peptides, proteins and nucleic acids.

DNA chips contained in cartridges of the above mentioned type have a wide range of applications. For example, they may be used for studying the structure-activity relationship between different biological materials or determining the DNA-sequence of an unknown biological material. For instance, the DNA-sequence of such unknown material may be determined by, for example, a process known as sequencing by hybridization. In one method of sequencing by hybridization, sequences of diverse materials are formed at known locations on a surface of a chip, and a solution containing one or more targets to be sequenced is applied to that surface. The targets will bind or hybridize with only complementary sequences on the substrate. The locations at which hybridization occurs are detected with appropriate detection systems by labeling the targets with a fluorescent dye, radioactive isotope, enzyme, or other marker. Information about target sequences can be extracted from the data obtained by such detection systems.

By combining various available technologies, such as photolithography and fabrication techniques, substantial progress has been made in the fabrication and placement of diverse materials on chips of the above mentioned kind. For example, thousands of different sequences may be fabricated on a single substrate of about 1.28 square centimeter in only a small fraction of the time required by conventional methods. Such improvements make these substrates practical for use in various applications, such as biomedical research, clinical diagnostics, and other industrial markets, as well as the emerging field of genomics, which focuses on determining the relationship between genetic sequences and human physiology.

The chip is inserted into a wall of a one-way cartridge with its active surface facing the interior of the so-called process chamber within the cartridge.

In the above mentioned method of sequencing by hybridization, processing of the coating on the active surface of the chip includes flooding of the process chamber of the cartridge with a solution containing one or more targets to be sequenced.

For several applications, e.g. the so called dynamic hybridization, a good mixing of the liquid sample and the reactants on the active surface of the chip shaped carrier is required. Experiments show that such a good mixing cannot be achieved by known methods like the above-mentioned.

A further drawback of prior art chambers is that a complete removal of liquid contained in the process chamber is difficult to achieve with the configuration and dimensions of prior art chambers, although this is necessary because during the analysis process not only the liquid sample to be analyzed, but also other liquids containing different substances are introduced into the process chamber in various process steps and each of those liquids should be completely removed after each process step.

In known prior art devices the liquid sample is supplied to the process chamber of the cartridge via a valve block which is connected by a conduit to an inlet port of the cartridge. This prior art arrangement has two serious drawbacks. On the one hand air bubbles in the valve block and/or the connecting conduit get into the process chamber, prevent that the entire active surface of the chip is accessible to the liquid sample to be examined, and prevent thereby obtaining reliable test results. On the other hand use of one and the same valve block over longer periods of time and connection of the same valve block to different process chambers raises the problem of contamination by carry-over of the liquid samples to be tested, and this is a serious obstacle in a process having as main aim obtaining reliable test results. Clogging of the valves due e.g. to high salt concentration of liquids being processed is a further drawback which negatively affects the reliability of the operation of the analysis system. Moreover when several cartridges have to be processed in parallel, a plurality of conduits and a complex and therefore expensive valve block is required in order to supply the liquid samples to be tested to the cartridges.

### SUMMARY OF THE INVENTION

A first aim of the invention is to provide a method of the above mentioned kind which provides an efficient mixing of the liquid sample with the reactants on the active surface of the chip shaped carrier. According to a first aspect of the invention this aim is achieved by means of a method defined by claim 1.

A second aim of the invention is to provide a system of the above mentioned kind which makes possible to perform a method according to the invention at low cost. According to a second aspect of the invention this aim is achieved by means of a system defined by claim 5.

A third aim of the invention is to provide a reaction vessel of the above mentioned kind which makes possible to perform a method according to the invention at low cost and which in addition allows a complete removal of liquid from the reaction vessel and thereby from the process chamber where the chip shaped carrier is located. According to a third aspect of the invention this aim is achieved by means of a reaction vessel defined by claim 9.

The main advantages provided by the invention are as follows:
- The shape of the process chamber within the reaction vessel makes possible to achieve a very efficient mixing effect even when smaller chips are used and this advantage is attained in particular because the chamber has a geometrical configuration and dimensions which are more favorable for this purpose than those of prior art chambers.
- The shape of the process chamber within the reaction vessel and the relative position of the chip shaped carrier within that chamber make possible to remove almost entirely liquid contained in that chamber and thereby satisfy high requirements in this respect.
- Since the all liquids required for performing the analysis methods are provided to the process chamber or removed therefrom by respective pipetting operations, the above-mentioned drawbacks related to the use of valve blocks are eliminated.

### BRIEF DESCRIPTION OF THE DRAWINGS

The subject invention will now be described in terms of its preferred embodiments with reference to the accompanying drawings. These embodiments are set forth to aid the understanding of the invention, but are not to be construed as limiting.
Fig. 1 shows a perspective view of a reaction vessel 11 according to the invention
Fig. 2 shows a perspective exploded view of the reaction vessel 11 shown by Fig. 1.
Fig. 3 shows a top view of the reaction vessel 11 shown by Fig. 1.
Fig. 4 shows a front view of the reaction vessel 11 shown by Fig. 1.
Fig. 5 shows a cross-sectional front view of the reaction vessel 11 along line A-A in Fig. 3.
Fig. 6 shows a cross-sectional front view of the reaction vessel 11 along line B-B in Fig. 3.
Fig. 7 shows a cross-sectional side view of the reaction vessel 11 along line C-C in Fig. 4.
Fig. 8 shows a cross-sectional top view of the reaction vessel 11 along line D-D in Fig. 4.
Fig. 9 shows a cross-sectional bottom view of the reaction vessel 11 along line E-E in Fig. 4.
Fig. 10 shows a cross-sectional side view of the reaction vessel 11 along line F-F in Fig. 4.
Fig. 11 shows a cross-sectional, exploded view of means used according to the invention for mounting a chip shaped carrier 21 in a side wall 16 of a reaction vessel 11.
Fig. 12 shows a cross-sectional view of the means represented in Fig. 11 after they are assembled according to the invention.
Fig. 13 shows a cross-sectional, exploded view of means used according to the invention for mounting a chip shaped carrier 21 in a side wall 16 of a reaction vessel 11.
Fig. 14 shows the same as Fig. 13, but with a chip inserted and energy sources
Fig. 15 shows a cross-sectional view of the means represented in Fig. 13 after chip shaped carrier 21 has been mounted in a side wall 16.
Fig. 16 shows a top view of the reaction vessel 11 shown by Fig. 1 and a vessel holder 71 as well as an example of a trajectory 72 of the reaction vessel for achieving a mixing effect.
Fig. 17 shows a cross-sectional side view of the reaction vessel 11 similar to Fig. 7 but shows in addition a cap 51 for closing vessel 11.
Fig. 18 shows a perspective view of a gripper 62 for interacting with a cap 51 of reaction vessel 11 for removing that cap from the vessel, closing the vessel and/or transporting the cap and/or the reaction vessel.
Fig. 19 shows a perspective view of a transport device 61 for transporting gripper 62 in three directions X, Y, Z normal to each other.
Fig. 20 shows a perspective exploded view of the components of gripper 62 in Figures 18 and 19.

### REFERENCE NUMERALS IN DRAWINGS

- 11: reaction vessel
- 12: tubular body
- 13: bottom wall
- 14: upper opening
- 15: side wall
- 16: side wall
- 17: process chamber
- 18: transparent zone of side wall 15
- 19: thermal interface
- 20 21: chip shaped carrier
- 22: active surface of chip shaped carrier
- 23: outer surface of carrier 21 opposite to active surface
- 22 24 25 26: inner surface of side wall 16
- 27: outer surface of side wall 16
- 28 29 30 31: opening in side wall 16
- 32: cavity
- 33: bottom surface
- 34: wall surface
- 35: opening
- 36: sealing frame
- 37: gap
- 38: cavity
- 39: locking frame
- 40 41: liquid
- 42: free surface of liquid
- 43 44 45 46 47 48: cavity
- 49: cavity
- 50: joint clearance
- 51: cap
- 52 53: bottom surface
- 54: wall surface
- 55: opening
- 56: hotmelt material
- 57: edge of chip shaped carrier 21
- 58 59: inner surface of hotmelt material layer
- 60: laser light
- 61: transport mechanism
- 62: gripper
- 63: pin at end part of gripper 62
- 64: annular recess in cap 51
- 65: annular recess in cap 51
- 66 67 68 69 70 71: vessel holder
- 72: trajectory of vessel holder
- 73 74: electro-optical measuring device
- 75: electro-optical measuring device

### DETAILED DESCRIPTION OF PREFERRED EXAMPLES

### EXAMPLE OF A REACTION VESSEL ACCORDING TO THE INVENTION

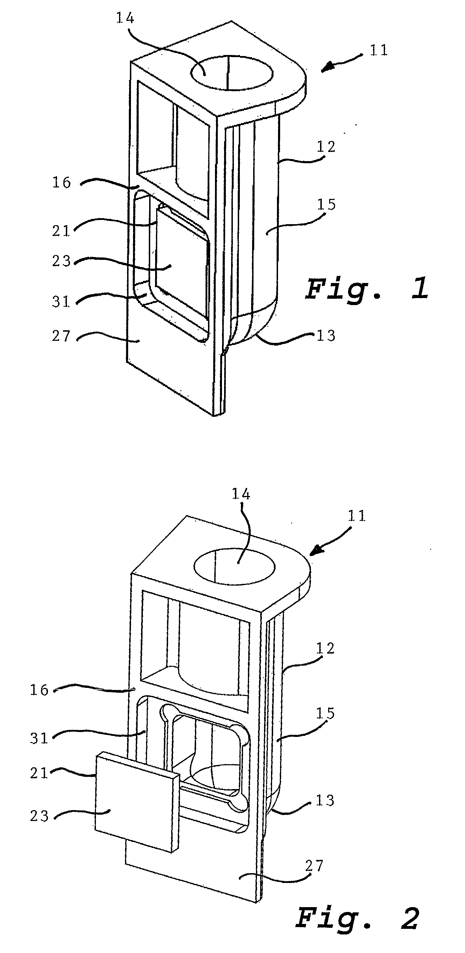

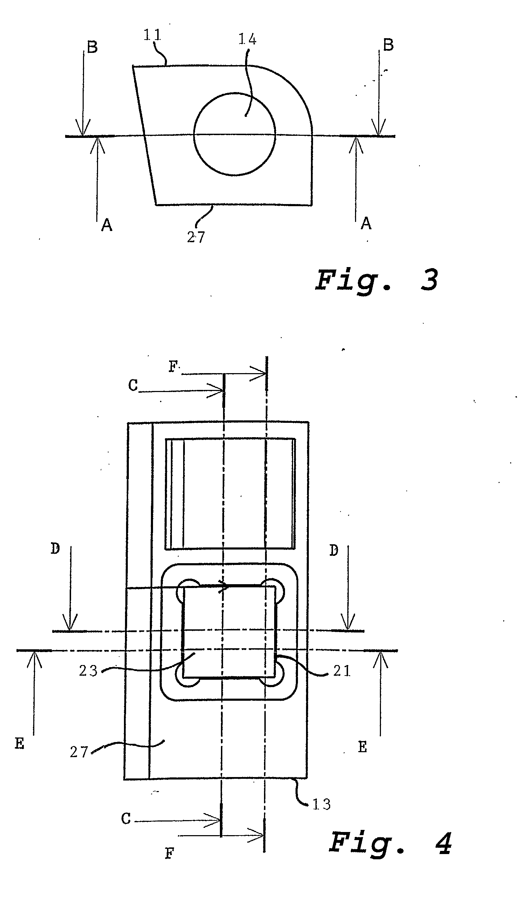

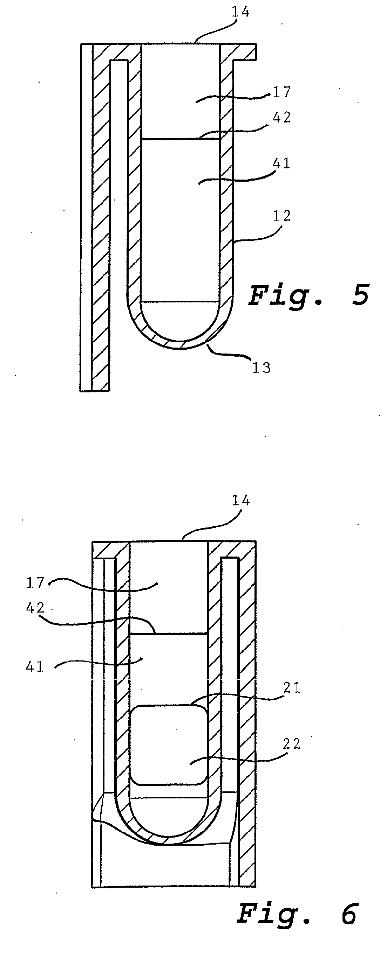

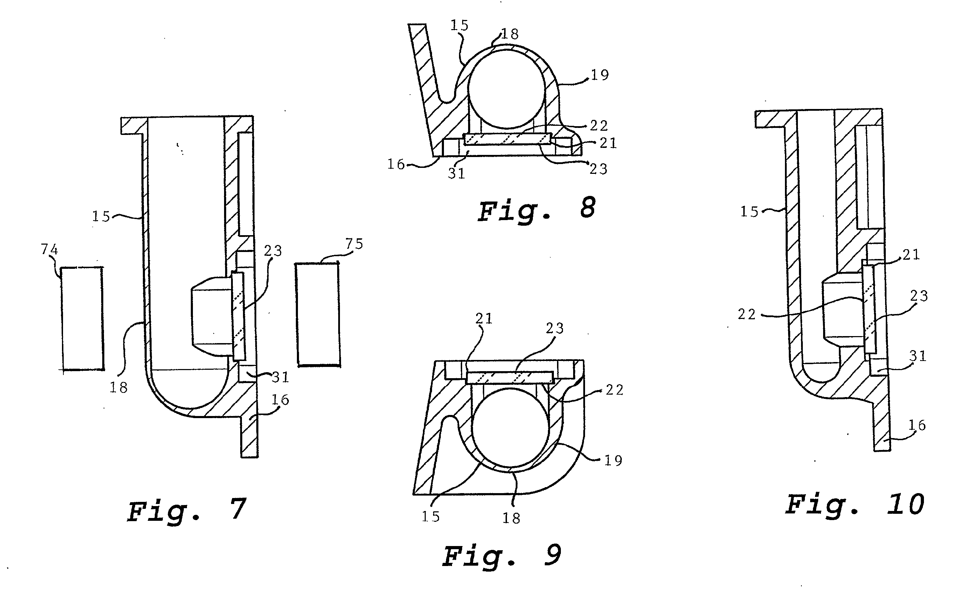

As shown by Figures 1-10 a reaction vessel 11 according to the invention comprises a tubular body 12 which has a bottom wall 13, an upper opening 14 and side walls 15, 16 which extend between bottom wall 13 and upper opening 14. Bottom wall 13 and side walls 15, 16 form a process chamber 17 for receiving a liquid 41 to be processed. This liquid is e.g. a liquid sample to be analyzed or other liquids used in various steps of the analysis process.

In contrast to prior art process chambers for carrying out similar processes, liquid 41 can only be introduced into and removed from process chamber 17 through the upper opening 14 of tubular body 12.

Reaction vessel 11 further comprises a chip shaped carrier 21 which has an active surface 22 formed by an array of biological polymers. Active surface 22 is accessible to a liquid 41 contained in process chamber 17. Chip shaped carrier 21 is located in an opening 31 of a side wall 16 of tubular body 12 or on the inner surface of side wall 16 or in a recess formed in the inner surface of side wall 16. This particular location of the chip shaped carrier is advantageous because it allows removing entirely any liquid contained in reaction vessel by a simple pipetting operation during which a pipetting tip is inserted into the vessel until it practically touches the bottom of the vessel. Since the chip shaped carrier and the active surface thereof are not at all in the travel path of the pipetting tip this tip cannot cause any damage of the active surface of the chip shaped carrier.

Two examples of means for mounting chip shaped carrier 21 in an opening 31 of a side wall of vessel 11 are described below.

In a preferred embodiment the tubular body 12 of reaction vessel 11 is so configured and dimensioned that process chamber 17 is adapted to receive a predetermined amount of liquid 41 and that when process chamber 17 contains a predetermined amount of liquid 41 and is at rest there is an air space between the free surface 42 of the liquid 41 and upper opening 14 and the entire surface of active surface 22 is in contact with the liquid 41 contained in process chamber 17.

In a preferred embodiment the chip shaped carrier 21 is located at a predetermined distance from the bottom wall 13 and from upper opening 14 of tubular body 12.

In a preferred embodiment the chip shaped carrier 21 is transparent and thereby enables performing electro-optical measurements of the active surface 22 of chip shaped carrier 21.

In a preferred embodiment tubular body 12 has a side wall 15 located substantially in face of the active surface 22 of chip shaped carrier 21 and side wall 15 has a transparent zone 18 which enables performing electro-optical measurements of the active surface 22 of chip shaped carrier 21.

In a preferred embodiment tubular body 12 comprises a thermal interface 19 adapted to be put in contact with a heat transfer element located outside of reaction vessel 11. Thermal interface 19 thereby enables heating and cooling of the contents of reaction vessel 11 by means of the heat transfer element. Thermal interface 19 is preferably a zone of a side wall 15 of tubular body 12.

In a preferred embodiment chip shaped carrier 21 is located in an opening 31 of one of a side wall 16 of tubular body 12 and has an outer surface 23 which is adapted to be contacted by a heat transfer element located outside of the vessel 11.

Tubular body 12 is made e.g. by injection molding of a plastic material suitable for satisfying on the one hand the thermal requirements of the process to be carried out and on the other hand the optical requirements for allowing electro-optical measurements of the active surface 12 of chip shaped carrier 21.

In a preferred embodiment process chamber 17 has an inner width larger than 1.5 millimeter at least in the region of reaction vessel 11 over which the active surface 22 of the chip shaped carrier 21 extends.

In a preferred embodiment tubular body 12 is so configured and dimensioned that process chamber 17 is adapted to receive a predetermined amount of liquid 41 which lies in a range going from 10 to 800 microliters.

In a preferred embodiment tubular body 12 is so configured and dimensioned that process chamber 17 has approximately the shape of a cuboid having sides lengths which are equal or of the same order of magnitude. That cuboid has e.g. a side length of about 3 millimeter or larger than 3 millimeter. This shape of process chamber 17 distinguishes it from prior art processing chambers for a similar purpose and is particularly advantageous because it allows performing a very effective vortex mixing.

In a preferred embodiment the active surface 22 of chip shaped carrier 21 has the shape of a square and the side length of this square lies in a range going from 2 to 10 millimeter.

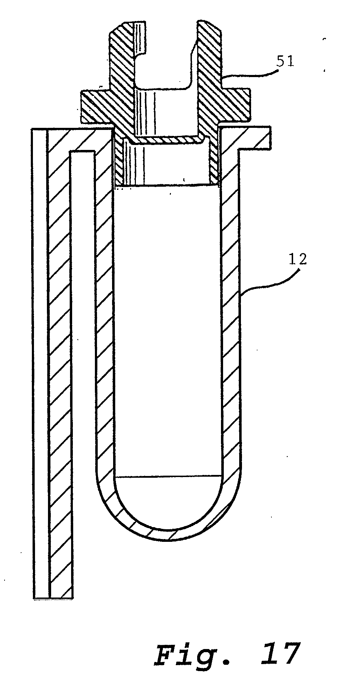

In a preferred embodiment shown by Fig. 17 reaction vessel 11 further comprises a cap 51 for closing upper opening 14 of tubular body 12, and cap 51 is a removable closure of opening 14.

In a preferred embodiment cap 51 is so configured and dimensioned that a part thereof is a transport interface adapted to cooperate with a gripper 62 of a transport mechanism 61. Cooperation of the gripper 62 and the cap 51 enables automatic transport of the vessel 11 by means of transport mechanism 61.

### FIRST EXAMPLE OF MEANS FOR MOUNTING A CHIP SHAPED CARRIER 21 IN AN OPENING OF A SIDE WALL OF REACTION VESSEL 11

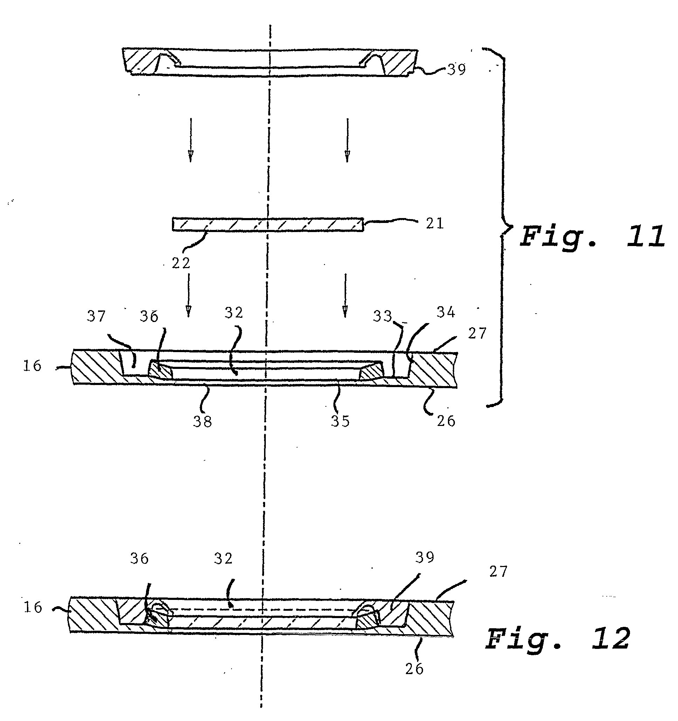

Figures 11 and 12 show a portion of side wall 16 into which a chip shaped carrier 21 is inserted in an opening 31 of side wall 16. The means for fixing carrier 21 in opening 31 described hereinafter provide a liquid- and gas-tight connection between the chip shaped carrier and side wall 16. The fixing method and means described hereinafter are based on the method and means described in U.S. Patent Application with publication number US 2002/0019044 A1 the contents of which is incorporated herein by reference.

As can be appreciated from Fig. 11, sidewall 16 has an inner surface 26 and outer surface 27, and opening 31 defines a first cavity 32 for receiving a chip shaped carrier 21 and a second cavity 38 which faces the interior of process chamber 17 within reaction vessel 11.

The part of cavity 32, which as shown in Fig. 12 lies between chip shaped carrier 21 and the plane defined by outer surface 27, defines the numeric aperture available for emission of fluorescence light. This aperture defines the optical accessibility of the chip which has to be guaranteed for a reading out.

Chip 21 is e.g. made of glass, has a thickness of e.g. 0.7 or 1.0 millimeter, and has substantially the shape of a square. Since the size of chip 21 has a relatively high tolerance of e.g. 0.0762 millimeter, in the embodiment described hereinafter the space available in cavity 32 for receiving and positioning chip 21 has a corresponding joint clearance.

Cavity 32 has a flat or substantially flat bottom surface 33 and inclined side wall surfaces 34 which extend between outer surface 27 of side wall 16 and bottom surface 33. Each of the inclined side wall surfaces 34 forms an obtuse angle with bottom surface 33. Bottom surface 33 has an opening 35 which opens into second cavity 38.

As can be appreciated in particular from Figures 11 and 12 this embodiment offers the advantage that it allows insertion of chip shaped carrier 21 into its position in cavity 32 from the outside of reaction vessel 11.

A sealing frame 36, which is made of a compressible material, is part of side wall 16 and is connected to bottom surface 33 of cavity 32. In a preferred embodiment, sealing frame 36 is formed onto bottom surface 33 by an injection molding process. In another embodiment sealing frame 36 is bound by adherence to bottom surface 33.

A locking frame 39 represented in Fig. 11 is used for tightly connecting chip shaped carrier 21 to side wall 16. The cross-section of locking frame 39 is wedge-shaped. In a preferred embodiment, locking frame 39 is apt to be bound to side wall 16 by a welding process.

As can be appreciated from Figures 11 and 12, chip 21 is positioned in cavity 32 of side wall 16.

As can be appreciated from Fig. 12, the shape and dimensions of cavity 32, chip 21, sealing frame 36, locking frame 39 and opening 35 of bottom surface 33 of cavity 32 are so chosen that chip 21 fits into the space delimited by sealing frame 36, and a gap 37 exists between sealing frame 36 and the inclined side wall surfaces 34 of first cavity 32, and locking frame 39 is slightly larger than gap 37, but locking frame 39 is however insertable into gap 37 by a pressure exerted on locking frame 39 against side wall 16. That pressure causes a compression of sealing frame 36 and a corresponding pressure on a substantial part of the outer surface of the lateral periphery of chip 21. The latter outer surface is in contact with sealing frame 36.

In a preferred embodiment, side wall 16 and locking frame 39 are made of a first plastic material, e.g. a polypropylene, and sealing frame 36 is made of a second plastic material, e.g. a thermoplastic elastomer, which is softer than the first plastic material.

As can be appreciated from Fig. 12, a part of cavity 32 forms a window which provides visual and optical access to the active surface of chip shaped carrier 21.

As can be appreciated from Fig. 12, the above described means for attaching chip 21 to side wall 16 make it possible to mount chip 21 so that it is nearly coplanar with the side of side wall 16 which faces process chamber 17.

Since the chip is only held by friction forces, a minimum chip contact force of 5N has been defined to ensure proper operation, and in particular to ensure that the chip mounting remain liquid-tight up to an overpressure of 300 millibar.

### SECOND EXAMPLE OF MEANS FOR MOUNTING A CHIP SHAPED CARRIER 21 IN AN OPENING OF A SIDE WALL OF REACTION VESSEL 11

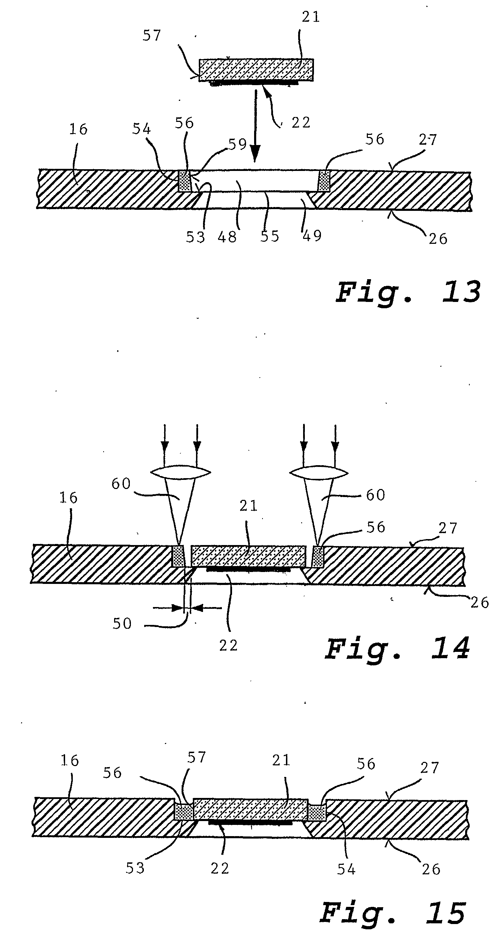

Figures 13, 14 and 15 show a portion of side wall 16 into which a chip shaped carrier 21 is inserted in an opening 31 of side wall 16. The means for fixing carrier 21 in opening 31 described hereinafter provide a liquid- and gas-tight connection between the chip shaped carrier and side wall 16. The fixing method and means described hereinafter are based on the method and means described in European Patent Application No. 02077768.6 and U.S. Patent Application No. 10/205,734 the contents of which are incorporated herein by reference.

As can be appreciated from Fig. 13, side wall 16 has an outer surface 27 and inner surface 26, a first cavity 48 for receiving a chip shaped carrier 21 and a second cavity 49 which forms a window providing visual and optical access to said first cavity 48 and thereby to the active surface 22 of chip shaped carrier 21.

Typically, chip 21 is made of glass, has a thickness of 0.7 or 1.0 millimeter, and has substantially the shape of a square. Since the size of chip 21 has a relatively high dimensional tolerance of e.g. 0.0762 millimeter of length and width, in the embodiment described hereinafter the space available in cavity 48 for receiving and positioning chip 21 has a corresponding joint clearance 50.

Cavity 48 has a flat bottom surface 53 and side wall surfaces 54 which extend between outer surface 27 of side wall 16 and bottom surface 53. As shown by Figures 13-15, a layer of a solid sealing hotmelt material 56 is arranged on side wall surfaces 54. The solid hotmelt is fusible by heating, specifically by irradiation with laser light, and solidifies again when cooled. In order to facilitate the insertion of the chip 21, the inner surfaces 59 of the hotmelt material layer 56 may be inclined so that an opening tapering to the bottom surface 53 is obtained. For this purpose, the tapering caused by injection molding of this piece may suffice.

The bottom surface 53 has an opening 55 which opens into second cavity 49.

As can be appreciated from Figures 13 and 14, chip 21 is positioned in cavity 48 of side wall 16. The hotmelt 56 is heated by means of laser light 60 provided by a suitable light source. The laser light is directed sequentially to a number of points of hotmelt material layer 56 or simultaneously to the whole hotmelt material layer 56. The heated hotmelt 56 becomes then fluid and fills the clearance 50 between walls 54 and the edge of the chip 21. Obviously, irregularities in the shape of the edge of the chip 21 do not have any sensible influence on this process, neither on the quality of the bond between the hotmelt 56 and the chip 21. Just on the contrary, it can be expected that irregularities ameliorate its mechanical strength.

Further advantages of the above method for fixing chip shaped carrier in side wall 16 are:
a) there is no mechanical stress involved in establishing the bond between side wall 16 and chip 21 in contrast to known devices where the chip is held by clamping means;
b) no adhesive has to be administered after positioning the chip, and the disadvantage of the known adhesives set forth in the introduction are avoided;
c) the chip may be inserted from the outer surface of side wall 16;
d) solidification of the hotmelt, i.e. the bonding process, is a physical process (phase transition), and quite fast;
e) the hot melt material may preferably be chosen such that it retains permanently a certain elasticity;
f) the hotmelt material does not impair fluorescence measurements, i.e. has low fluorescence activity at 633 nm; and
g) increased life time with respect to conventional adhesives.

In one embodiment, the following materials were used:
- Chip 21: glass
- Hotmelt layer 56: Ecomelt P1 Ex318 (Collano Ebnöther AG, Schweiz):
   Softening temperature: 90 °C (DIN 52011; ASTM D36/E28); working temperature range: 150-180 °C, typically 160 °C;

It has been experimentally verified that the chip is safely held against an overpressure of 500 mbar at 20 °C, and that no leakage occurs. Even at 60 °C, the joint withstands the pressure for some minutes.

Fig. 15 shows the fixed state of chip shaped carrier 21 in a cross-sectional view. Fig. 15 shows in particular that the hotmelt 56 fills up the clearance 50 from the bottom.

As can be appreciated from Figures 13 to 15, the shape and dimensions of cavity 48, chip 21, hotmelt layer 56 and opening 55 of bottom surface 53 of cavity 48 are so chosen that chip shaped carrier 21 fits into the space delimited by hotmelt layer 56.

### EXAMPLE OF A SYSTEM ACCORDING TO THE INVENTION

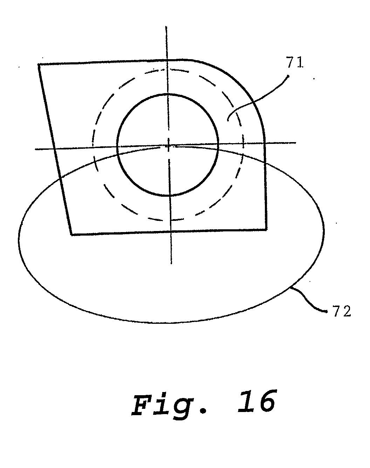

According to the invention, a system for processing a biological sample contained in a liquid comprises a reaction vessel 11 of the type described above, a vessel holder 71 for holding reaction vessel 11 and means for moving vessel holder 71 and thereby vessel 11 along a predetermined trajectory 72, which can be e.g. as shown by Fig. 16, for causing a relative motion of liquid 41 contained in process chamber 17 with respect to the active surface 22 of chip shaped carrier 21. In order to achieve trajectory 72 of vessel holder 71 the system preferably comprises a vortexing motor (not shown) and suitable mechanical transmission means. The path of trajectory 72 can differ from the path shown as example in Fig. 16 and can be any path suitable for achieving an effective mixing effect.

As described above, reaction vessel 11 comprises a tubular body 12 which has a bottom wall 13, an upper opening 14 and side walls 15, 16 which extend between bottom wall 13 and upper opening 14. Bottom wall 13 and side walls 15, 16 form a process chamber 17 for receiving a liquid 41 to be processed. This liquid is e.g. a liquid sample to be analyzed or other liquids used in various steps of the analysis process.

In contrast to prior art process chambers for carrying out similar processes, liquid 41 can only be introduced into and removed from process chamber 17 through the upper opening 14 of tubular body 12.

Reaction vessel 11 further comprises a chip shaped carrier 21 which has an active surface 22 formed by an array of biological polymers. Active surface 22 is accessible to a liquid 41 contained in process chamber 17. Chip shaped carrier 21 is located in an opening 31 of a side wall 16 of tubular body 12 or on the inner surface of side wall 16 or in a recess formed in the inner surface of side wall 16.

In a preferred embodiment the system further comprises a heat transfer element for heating and cooling of the contents of reaction vessel 11. The heat transfer element is located outside of the reaction vessel 11 and is adapted to be put in contact with a thermal interface 19 which is part of tubular body 12 of reaction vessel 11. Thermal interface 19 is preferably a zone of a side wall 15 of tubular body 12.

In a preferred embodiment of the system, chip shaped carrier 21 is located in an opening 31 of a side wall 16 of tubular body 12 and has an outer surface 23 adapted to be contacted by a heat transfer element located outside the reaction vessel 11, and the system further comprises a heat transfer element for heating and cooling of the contents of the reaction vessel 11. The heat transfer element (not shown) is located outside of the reaction vessel 11 and is adapted to be put in contact with the outer surface 23 of chip shaped carrier 21.

In a preferred embodiment the system further comprises an electro-optical measuring device 74 for examining the active surface 22 through transparent zone 18 of side wall 15 or an electro-optical measuring device 75 for examining the active surface 22 through a transparent zone of chip shaped carrier 21. Electro-optical measuring device 74 respectively 75 is e.g. a fluorometer.

A preferred embodiment of the system further comprises an automatic pipetting device for effecting pipetting operations necessary to introduce the necessary liquids into reaction vessel 11 or to remove liquids from the vessel. Such automatic pipetting device may include transport means for bringing a pipetting tip to selected pipetting positions. Such transport means may be of the type adapted for moving a pipetting tip in three directions X, Y, Z which are normal to each other.

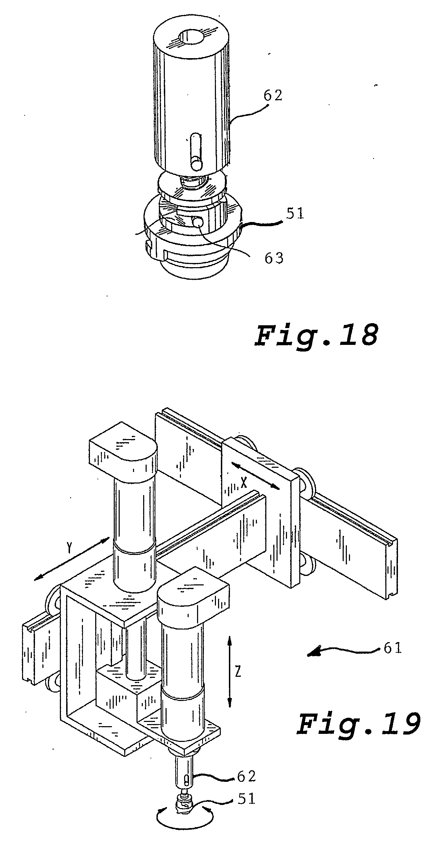

A preferred embodiment of the system further comprises a gripper 62 of the type shown by Fig. 18 and a transport mechanism 61 shown by Fig. 19 for moving and actuating gripper 62 for effecting one or more of the following operations:
- removing a cap 51 from a reaction vessel 11,
- replacing a removed cap 51 into the upper opening 14 of a reaction vessel,
- picking up a cap 51 of a reaction vessel and the reaction vessel connected thereto and bringing both from a first position to a second position.

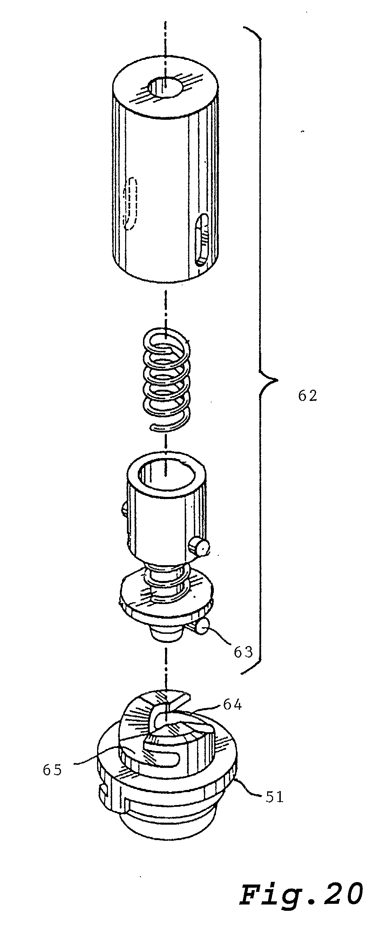

As shown by Fig. 20 gripper 62 is so configured and dimensioned that the lower end part thereof is adapted to cooperate with a corresponding part of cap 51 and form a removable connection therewith. For this purpose the end part of the gripper has pin shaped projections 63 that enter and engage annular recesses 64 and 65 respectively in the top part of cap 51 for forming a connection which can be locked by rotating gripper 62 in one sense and unlocked by rotating gripper 62 in the opposite sense. Cooperation of the gripper 62 and the cap 51 thus enables automatic transport of the vessel 11 by means of transport mechanism 61 shown by Fig. 19.

The operation of a gripper of the above mentioned type and its cooperation with a cap is described in detail in U.S. Patent Specification No. 6,216, 340 B1 the contents of which is incorporated herein by reference.

### EXAMPLE OF A METHOD ACCORDING TO THE INVENTION

According to the invention, a method for processing a biological sample contained in a liquid comprises the following steps:
(a) introducing a liquid 41 into a process chamber 17 of a reaction vessel 11 which comprises
   a tubular body 12 which has a bottom wall 13, an upper opening 14 and side walls 15, 16 which extend between bottom wall 13 and upper opening 14,
   bottom wall 13 and side walls 15, 16 forming process chamber 17, and
   a chip shaped carrier 21 having an active surface 22 which is formed by an array of biological polymers, said active surface 22 being accessible to liquid 41 contained in said process chamber 17,
   chip shaped carrier 21 being located in an opening 31 of a side wall 16 of tubular body 12 or on the inner surface of a side wall 16 or in a recess formed in the inner surface of side wall 16,
(b) positioning reaction vessel 11 in a vessel holder 71, the latter positioning being effected before or after introduction of said liquid 41 into process chamber 17, and
(c) moving vessel holder 71 along a predetermined trajectory 72 for causing a relative motion of liquid 41 contained in process chamber 17 with respect to the active surface 22 of chip shaped carrier 21.

Within the scope of the invention the step of moving the vessel holder 71 along a predetermined trajectory includes any suitable method for agitating liquid contained in vessel 11 and thereby achieving an effective mixing of the liquid with reactants on the active surface 22 of chip shaped carrier or with any other reactants contained in vessel 11.

In a preferred embodiment of the method the vessel holder and thereby the reaction vessel are moved along a trajectory suitable for achieving a vortex mixing effect and said movement is preferably performed periodically with a predetermined frequency. The latter frequency is preferably higher than 1 cycle per second.

In a preferred embodiment introduction of liquids into and removal of liquids from reaction vessel 11 is carrier out exclusively by pipetting operations performed preferably by an automatic pipettor using pipetting tips which are introduced into vessel 11 for performing the pipetting operations. This procedure contributes to eliminate the risk of the presence of bubbles in processing chamber 17 of vessel 11.

Although a preferred embodiment of the invention has been described using specific terms, such description is for illustrative purposes only, and it is to be understood that changes and variations may be made without departing from the spirit or scope of the following claims.

## Claims

1. A method for processing a biological sample contained in a liquid, said method comprising
(a) introducing said liquid into a chamber (17) of a reaction vessel (11) which comprises
a tubular body (12) which has a bottom wall (13), an upper opening (14) and side walls (15, 16) which extend between said bottom wall (13) and said upper opening (14),
said bottom wall (13) and said side walls (15, 16) forming said chamber (17), and
a chip shaped carrier (21) having an active surface (22) which is formed by an array of biological polymers, said active surface being accessible to liquid (41) contained in said chamber (17),
said chip shaped carrier (21) being located in an opening (31) of a side wall (16) of said tubular body (12) or on the inner surface of said side wall (16) or in a recess formed in the inner surface of said side wall (16),
(b) positioning said reaction vessel (11) in a vessel holder (71), said positioning being effected before or after introduction of said liquid into said chamber (17), and
(c) moving said vessel holder (71) along a predetermined trajectory (72) for causing a relative motion of the liquid (41) contained in said chamber (17) with respect to said active surface (22) of said chip shaped carrier (21).

2. A method according to claim 1, wherein said moving is performed along a trajectory (72) suitable for achieving a vortex mixing effect.

3. A method according to claim 1, wherein said moving is performed periodically with a predetermined frequency.

4. A method according to claim 3, wherein said frequency is higher than 1 cycle per second.

5. A system for processing a biological sample contained in a liquid, said system comprising
(a) a reaction vessel (11) which comprises
a tubular body (12) which has a bottom wall (13), an upper opening (14) and side walls (15, 16) which extend between said bottom wall (13) and said upper opening (14),
said bottom wall (13) and said side walls (15, 16) forming said chamber (17), and
a chip shaped carrier (21) having an active surface (22) which is formed by an array of biological polymers, said active surface being accessible to liquid (41) contained in said chamber (17),
said chip shaped carrier (21) being located in an opening (31) of a side wall (16) of said tubular body (12) or on the inner surface of said side wall (16) or in a recess formed in the inner surface of said side wall (16),
(b) a vessel holder (71) for holding said reaction vessel (11), and
(c) means for moving said vessel holder (71) along a predetermined trajectory (72) for causing a relative motion of the liquid (41) contained in said chamber (17) with respect to said active surface (22) of said chip shaped carrier (21).

6. A system according to claim 5, which further comprises a heat transfer element for heating and cooling of the contents of the reaction vessel (11), said heat transfer element being located outside of the reaction vessel (11) and being adapted to be put in contact with a thermal interface (19) which is part of the tubular body (12) of the reaction vessel (11).

7. A system according to claim 6, wherein said thermal interface (19) is a zone of a side wall (15) of said tubular body (12).

8. A system according to claim 5, wherein said chip shaped carrier (21) is located in an opening (31) of a said wall of said tubular body (12) and has an outer surface (23) adapted to be contacted by a heat transfer element located outside the reaction vessel (11), said system further comprising a heat transfer element for heating and cooling of the contents of the reaction vessel (11), said heat transfer element being located outside of the reaction vessel (11) and being adapted to be put in contact with said outer surface (23) of said chip shaped carrier (21).

9. A reaction vessel for processing a biological sample contained in a liquid, said reaction vessel comprising
(a) a tubular body (12) which has a bottom wall (13), an upper opening (14) and side walls (15, 16) which extend between said bottom wall (13) and said upper opening (14),
said bottom wall (13) and said side walls (15, 16) forming a chamber (17) for receiving a liquid (41) to be processed, and
(b) a chip shaped carrier (21) having an active surface (22) which is formed by an array of biological polymers, said active surface being accessible to liquid (41) contained in said chamber (17),
said chip shaped carrier (21) being located in an opening (31) of a side wall (16) of said tubular body (12) or on the inner surface of said side wall (16) or in a recess formed in the inner surface of said side wall (16).

10. A reaction vessel according to claim 9, wherein said tubular body (12) is so configured and dimensioned that said chamber (17) is adapted to receive a predetermined amount of liquid (41) and that when said chamber (17) contains said amount of liquid and is at rest
there is an air space between the free surface (42) of the liquid (41) and said upper opening (14) and
the entire surface of said active surface (22) is in contact with the liquid (41) contained in said chamber (17).

11. A reaction vessel according to claim 9, wherein the chip shaped carrier (21) is located at a predetermined distance from the bottom wall (13) and from the upper opening (14) of said tubular body (12).

12. A reaction vessel according to claim 9, wherein said chip shaped carrier (21) is transparent and thereby enables performing electro-optical measurements of said active surface (22) of said chip shaped carrier (21).

13. A reaction vessel according to claim 9, wherein said tubular body (12) has a side wall (15) located substantially in face of said active surface (22) of said chip shaped carrier (21), said side wall (15) having a transparent zone (18) which enables performing electro-optical measurements of said active surface (22) of said chip shaped carrier (21).

14. A reaction vessel according to claim 9, wherein said tubular body (12) comprises a thermal interface (19) adapted to be put in contact with a heat transfer element located outside of the reaction vessel (11), thereby enabling heating and cooling of the contents of the reaction vessel by means of said heat transfer element.

15. A reaction vessel according to claim 14, wherein said thermal interface (19) is a zone of a side wall (15) of said tubular body (12).

16. A reaction vessel according to claim 9, wherein said chip shaped carrier (21) is located in an opening (31) of a side wall (16) of said tubular body (12) and has an outer surface (23) which is adapted to be contacted by a heat transfer element located outside of the reaction vessel (11).

17. A reaction vessel according to claim 9, wherein said chamber (17) has an inner width larger than 1.5 millimeter at least in the region of the reaction vessel (11) over which the active surface (22) of the chip shaped carrier (21) extends.

18. A reaction vessel according to claim 9, wherein said tubular body (12) is so configured and dimensioned that said chamber (17) is adapted to receive a predetermined amount of liquid lying in a range going from 10 to 800 microliter.

19. A reaction vessel according to claim 9, wherein said tubular body (12) is so configured and dimensioned that said chamber (17) has approximately the shape of a cuboid having sides lengths which are equal or of the same order of magnitude.

20. A reaction vessel according to claim 19, wherein said cuboid has a side length of about 3 millimeter or larger than 3 millimeter.

21. A reaction vessel according to claim 9, wherein the active surface (22) of the chip shaped carrier (21) has the shape of a square and the side length of this square lies in a range going from 2 to 10 millimeter.

22. A reaction vessel according to claim 9, wherein said reaction vessel further comprises a cap (51) for closing said upper opening (14) of said tubular body (12), said cap being a removable closure of said opening.

23. A reaction vessel according to claim 22, wherein said cap (51) is so configured and dimensioned that a part thereof is a transport interface adapted to cooperate with a gripper (62) of a transport mechanism (61), cooperation of the gripper (62) and the cap (51) enabling automatic transport of the reaction vessel (11) by means of said transport mechanism (61).

24. A reaction vessel according to claim 9, wherein liquid can only be introduced into and removed from said chamber (17) through said upper opening (14) of said tubular body (12).
